(19) Europäisches Patentamt
European Patent Office
Office européen des brevets

(11) Numéro de publication: **0 091 387**
**A2**

(12) # DEMANDE DE BREVET EUROPEEN

(21) Numéro de dépôt: 83420058.6

(22) Date de dépôt: 30.03.83

(51) Int. Cl.³: C 07 C 143/72
A 01 N 41/06

(30) Priorité: 01.04.82 FR 8205899

(43) Date de publication de la demande:
12.10.83 Bulletin 83/41

(84) Etats contractants désignés:
DE FR NL SE

(71) Demandeur: RHONE-POULENC AGROCHIMIE
14-20, rue Pierre Baizet
F-69009 Lyon(FR)

(72) Inventeur: Borrod, Guy
38 bis rue des Granges
F-69005 Lyon(FR)

(72) Inventeur: Chene, Alain Les Terrasses de St. Rambert
12, Chemin de Montpellas
F-69009 Lyon(FR)

(74) Mandataire: Brachotte, Charles et al,
RHONE-POULENC AGROCHIMIE P.I.D. BP 9163
F-69263 Lyon Cedex 09(FR)

(54) **Nouveaux herbicides dérivés d'acides phénoxybenzoiques à groupe sulfonimide.**

(57) Herbicide de formule:

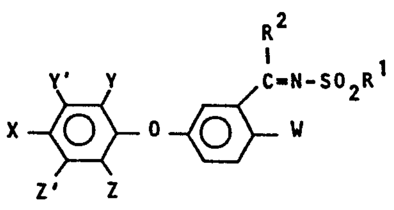

dans laquelle:

- W, Y, Y', X, Z', Z représentent l'atome d'hydrogène ou un atome d'halogène ou un groupe $NO_2$ ou CN, ou un groupe polyhaloalkyle, ou un groupe alkyle, ou alkoxyle,
- $R^1$ représente un groupe alkyle, alcényle, alcynyle ou phényle, éventuellement substitué,
- $R^2$ représente un atome d'halogène ou un groupe CN ou SCN ou $OR^3$ ou $SR^4$ ou $NR^5R^6$,
- $R^3$ représente un groupe alkyle éventuellement substitué ou un groupe allyle ou propargyle ou alkylcarbonyle ou alkylidène imino ou succinimido.
- $R^4$ représente l'atome d'hydrogène ou un cation ou a une des significations données pour $R^3$,
- $R^5$ représente l'atome d'hydrogène ou un groupe alkyle éventuellement substitué ou un groupe alkylcarbonyle ou alkyl sulfonyle.

EP 0 091 387 A2

L'invention se rapporte à des composés herbicides à groupe arylsulfonimide dérivés d'acides phénoxybenzoïques.

On connait l'acide 5-(2'-chloro-4'-trifluorométhyl phénoxy)-2-nitrobenzoïque, également appelé acifluorfen, et ses sels, ainsi que leur activité herbicide.

Dans le présent exposé, les produits chimiques ont été désignés selon la nomenclature française ; toutefois les chiffres indiquant les positions de substituants ont été placés avant les substituants concernés selon la nomenclature anglosaxonne.

On a en particulier proposé d'appliquer l'acifluorfen et ses sels sur des cultures de soja en traitement de postémergence pour contrôler des mauvaises herbes, spécialement des dicotylédones. Dans une telle application, les qualités requises pour un herbicide sont, à la dose considérée :

- la capacité de contrôler les mauvaises herbes majeures ou mauvaises herbes cibles,
- la sélectivité vis-à-vis du soja.

En essayant d'améliorer les propriétés herbicides de l'acifluorfen et ses sels, on a proposé de nombreux dérivés de ces composés notamment les esters d'alkyle, de cylcloalkyle, de thioalkyle, de phényle ainsi que les amides mono ou dialkylés. De tels composés sont décrits par les brevets américains US 3652645, 3784635, 3873302, 3983168, 3907866, 3798276, 3928416, 4063929 et d'autres. Les brevets européens 3416 et 23392 décrivent des sulfonamides dérivés des acides phénoxybenzoïques.

Un but de l'invention est de fournir des produits présentant une meilleure combinaison de propriétés herbicides en ce qui concerne l'activité sur les mauvaises herbes et la sélectivité sur les cultures. Un autre but de l'invention est de fournir des produits ayant une meilleure sélectivité pour des cultures autres que le soja, notamment les céréales.

L'invention concerne donc des produits de formule :

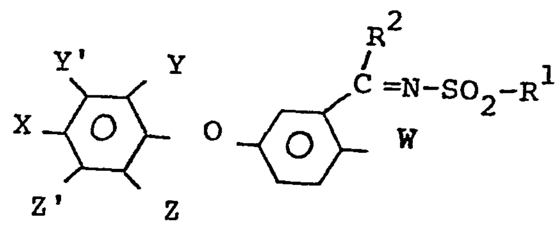

dans laquelle :

- W, Y, Y', X, Z', Z représentent l'atome d'hydrogène ou un atome d'halogène ou un groupe $NO_2$ ou CN ou un groupe polyhaloalkyle tel que $CF_3$ ou un groupe alkyle ou alkoxyle, les divers groupes alkyle ou alkoxyle précités, ayant le plus souvent de 1 à 4 atomes de carbone,
- $R^1$ représente un groupe alkyle, alcényle, alcynyle ou phényle, ce groupe étant éventuellement substitué,
- $R^2$ représente un atome d'halogène ou un groupe CN ou SCN ou $OR^3$ ou $SR^4$ ou $NR^5R^6$,
- $R^3$ représente un groupe alkyle éventuellement substitué ou un groupe allyle ou propargyle ou alkylcarbonyle ou alkylidène-imino ou succinimido,
- $R^4$ représente l'atome d'hydrogène ou un cation ou a une des significations données pour $R^3$,
- $R^5$ représente l'atome d'hydrogène ou un groupe alkyle éventuellement substitué ou un groupe alkylcarbonyle ou alkylsufonyle,
- $R^6$ a l'une des significations données pour $R^5$ ou représente un cation ou un groupe CN ou un groupe $OR^{10}$, $R^{10}$ étant l'atome d'hydrogène ou un cation, ou un groupe alkyle ou carboxylate ou carboxamide ou alkylcarbonyle.

Plus spécifiquement, ces radicaux sont le plus souvent choisis de manière que :

- W représente l'atome d'hydrogène ou un atome d'halogène, notamment Cl, Br, F ou un groupe $NO_2$ ou CN,
- Y représente l'atome d'hydrogène ou un atome d'halogène, notamment Cl, Br, F ou un groupe $NO_2$ ou CN ou $CF_3$ ou $CH_3$,
- Y', Z', Z représentent l'atome d'hydrogène ou un atome

d'halogène, notamment Cl, Br ou F,

- X représente un atome d'halogène, notamment Cl, Br, F ou un groupe $NO_2$ ou $CF_3$ ou $CH_3$ ou $C_2H_5$,

- $R^1$ représente :

. un groupe alkyle, ayant le plus souvent de 1 à 12 atomes de carbone et de préférence de 1 à 6 atomes de carbone, éventuellement substitué par :

+ un ou plusieurs atomes d'halogène, notamment Cl, Br, F ou par

+ un ou plusieurs groupes alkoxyle ou alkylthio ayant le plus souvent de 1 à 4 atomes de carbone, ou par

+ un ou plusieurs groupes CN, ou par

+ un groupe phényle, lui-même éventuellement substitué, notamment par un ou plusieurs atomes d'halogène, ou

. un groupe alcényle ou alcynyle ayant le plus souvent de 2 à 4 atomes de carbone, notamment un radical vinyle, éthynyle, allyle, propargyle, ou

. un groupe phényle, lui-même éventuellement substitué, notamment par un ou plusieurs atomes d'halogène, ou groupes nitro, ou radicaux alkyle ayant le plus souvent de 1 à 4 atomes de carbone.

- $R^2$ représente un atome d'halogène, de préférence le chlore, ou un groupe CN ou SCN, ou un groupe $OR^3$ ou $SR^4$ ou $NR^5R^6$.

- $R^3$ représente :

. un groupe alkyle, ayant le plus souvent de 1 à 4 atomes de carbone, et éventuellement substitué par :

+ un ou plusieurs atomes d'halogène, notamment Cl, Br, F, ou par

+ un ou plusieurs groupes alkoxyle ou alkylthio ayant le plus souvent de 1 à 4 atomes de carbone, ou par

+ un ou plusieurs groupes $NO_2$ ou CN, ou par

+ un groupe carboxyle ou l'un de ses dérivés de type sel, ester ou amide, notamment un groupe

$COOR^7$, $R^7$ étant l'atome d'hydrogène ou un groupe alkyle ayant le plus souvent de 1 à 4 atomes de carbone ou un cation métallique ou ammonium, ou par

+ un groupe alkylcarbonyle, notamment acétyle, ou par

+ un groupe phényle, lui-même éventuellement substitué, notamment par un ou plusieurs atomes d'halogène, ou par

+ un groupe 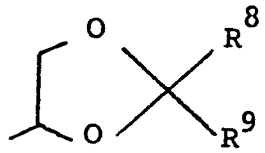

$R^8$ et $R^9$, identiques ou différents, étant l'atome d'hydrogène ou un groupe alkyle ayant le plus souvent de 1 à 4 atomes de carbone, ou

. un groupe allyle ou propargyle, ou

. un groupe alkylcarbonyle, notamment acétyle, ou

. un groupe 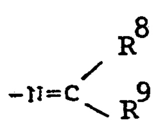 ou 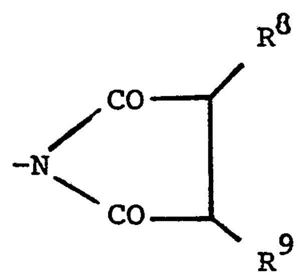

$R^8$ et $R^9$ ayant l'une des significations déjà indiquées.

- $R^4$ représente l'atome d'hydrogène, ou un cation de métal alcalin ou ammonium, ou a l'une des significations données pour $R^3$,

- $R^5$ représente :

. l'atome d'hydrogène, ou

. un groupe alkyle, ayant le plus souvent de 1 à 4 atomes de carbone, et éventuellement substitué par un groupe carboxyle ou l'un de ses dérivés de type sel, ester, ou amide, notamment un groupe $COOR^7$, $R^7$ ayant l'une des significations déjà indiquées, ou

. un groupe alkylcarbonyle ou alkylsulfonyle, notamment acétyle ou méthanesulfonyle.

- $R^6$ a l'une des significations données pour $R^5$, ou représente un cation de métal alcalin ou ammonium, ou un

groupe CN ou un groupe $OR^{10}$, $R^{10}$ étant :

+ l'atome d'hydrogène, ou

+ un cation de métal alcalin ou ammonium, ou

+ un groupe alkyle, ayant le plus souvent de 1 à 4 atomes de carbone, et éventuellement substitué par un groupe carboxyle ou l'un de ses dérivés de type sel, ester, ou amide, notamment un groupe $COOR^7$, $R^7$ ayant l'une des significations déjà indiquées, ou

+ un groupe $COOR^7$ ou $CONHR^7$, $R^7$ ayant l'une des significations déjà indiquées, ou

+ un groupe alkylcarbonyle, notamment acétyle.

Une sous-famille préférée selon l'invention est constituée par les produits de formule (I) dans laquelle W est $NO_2$, Y est Cl, Y' et Z' sont H, X est Cl ou $CF_3$, Z est Cl ou F ou de préférence H. Sont également particulièrement intéressants les composés dans lesquels $R^2$ est l'atome de chlore ou un groupe alkoxyle, ainsi que les composés dans lesquels $R^1$ et $R^3$ sont un radical alkyle, tandis que $R^4$ est l'atome d'hydrogène ou un groupe alkyle, que $R^5$ est l'atome d'hydrogène ou un groupe méthanesulfonyle, et que $R^6$ est l'atome d'hydrogène ou un cation de métal alcalin ou ammonium.

La formule (I) donnée ci-avant doit bien être comprise comme définissant les produits faisant l'objet de l'invention, mais ceux-ci peuvent présenter deux formes diastéréoisomères E et Z, en équilibre thermodynamique l'une avec l'autre, selon le schéma suivant :

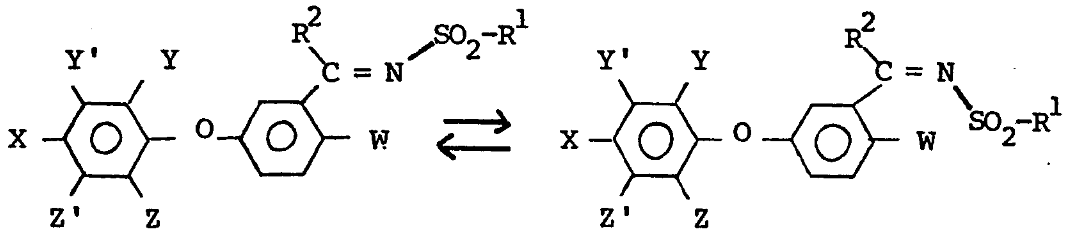

De plus, certains produits peuvent présenter des formes tautomères, et plus spécialement ceux dans la for-

mule desquels $R^4$ ou $R^5$ sont l'atome d'hydrogène, ce que montrent les schémas suivants :

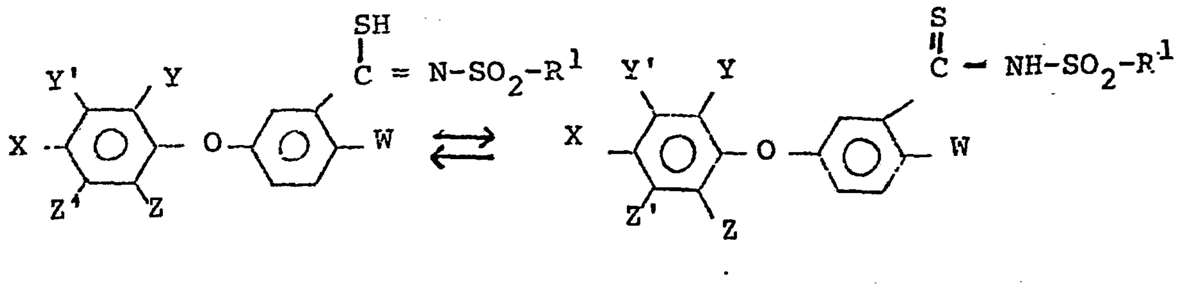

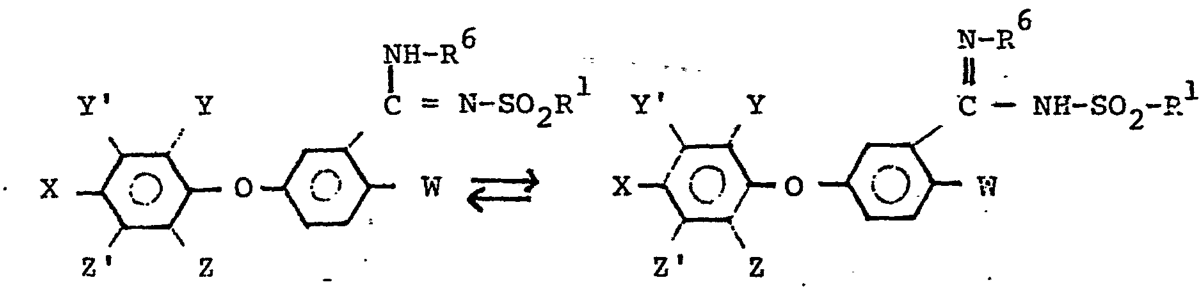

Ces formes diastéréoisomères et tautomères sont incluses dans l'invention au même titre que les formes physiquement distinctes qui résultent, par exemple, de différences de conformations, ou de liaisons hydrogène intra ou inter-moléculaires, ou d'autres phénomènes analogues.

L'invention concerne également un procédé de préparation des produits décrits plus haut.

Selon ce procédé, on fait réagir un composé de formule :

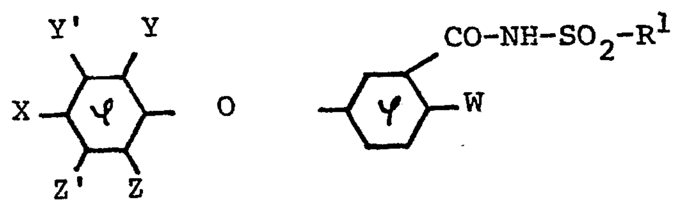
(II)

avec un agent d'halogénation de manière à obtenir un halogénure d'imidoyle de formule :

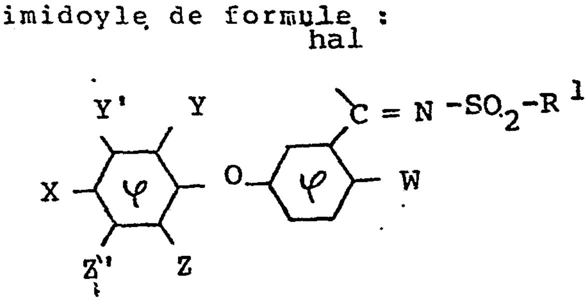
(III)

hal représentant un atome d'halogène, de préférence le chlore.

Comme agent d'halogénation on peut citer $COCl_2$, $SO_2Cl_2$, $(COCl)_2$, $PCl_5$, $PCl_3$, $POCl_3$, $SOCl_2$, et d'autres ; $COCl_2$ et $PCl_5$ sont préférés. La réaction s'effectue avantageusement entre - 30 et + 150°C dans un solvant, de préférence un hydrocarbure aliphatique ou aromatique, éventuellement halogéné tel que le dichlorométhane, le 1,2-dichloroéthane, le toluène, le chlorobenzène ; on opère en présence ou en absence d'un accepteur d'acide tel qu'une amine tertiaire (par exemple la triéthylamine ou la pyridine).

L'halogénure d'imidoyle de formule (III) est alors mis en réaction avec un composé de formule :

$$R^2\text{-H} \quad \text{(IV)}$$

($R^2$ ayant la signification donnée ci-avant sauf halogène), de manière à produire le composé de formule (I). La réaction s'effectue habituellement entre 0 et 150°C dans un solvant inerte et en présence d'un accepteur d'acide tel qu'une amine tertiaire (par exemple la triéthylamine ou la pyridine). Comme solvants, on peut citer les hydrocarbures aliphatiques ou aromatiques, éventuellement halogénés tel que le dichlorométhane, le 1,2-dichloroéthane, le toluène, ou les éthers, ou les nitriles.

Dans le cas où $R^2$ est $OR^3$, certains composés de formule (I) peuvent aussi être préparés selon la réaction :

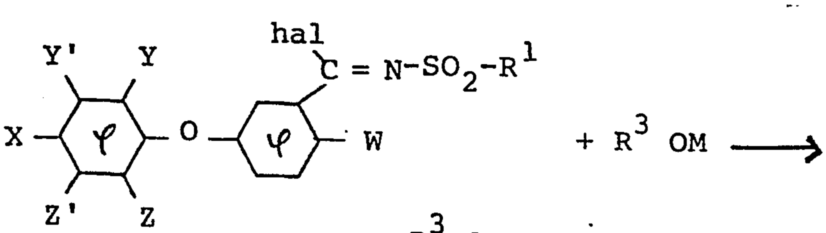

(IV)

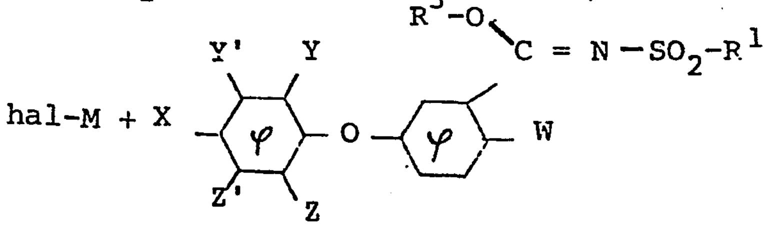

(V)

- M représentant l'atome d'hydrogène ou un atome de métal alcalin tel que le sodium ou le potassium.

Cette dernière réaction s'effectue avantageusement entre 10 et 150°C ; on peut aussi opérer dans un solvant constitué par un composé de formule $R^3OH$.

Dans le cas où $R^2$ est $SR^4$ ou $NR^5R^6$, certains composés de formule (I) peuvent aussi être préparés selon la réaction précédente, mais en remplaçant le réactif de structure $R^3$-OM par un réactif de structure $R^4$-SM ou $R^5R^6$-NM.

Ces deux dernières réactions s'effectuent avantageusement, entre 10 et 150°C, dans un solvant inerte tel qu'un hydrocarbure aliphatique ou aromatique, éventuellement halogéné comme le toluène, le xylène ou le chlorobenzène.

Dans le cas où $R^6$ est $OR^{10}$, certains composés de formule (I) peuvent aussi être préparés en mettant en réaction un composé de formule :

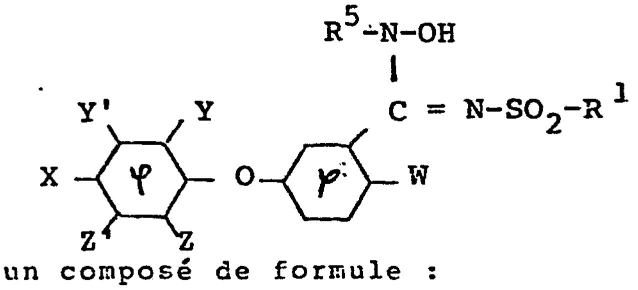

(VI)

avec un composé de formule :

$R^{10}$-hal (VII)

hal représentant un atome d'halogène. La réaction s'effectue habituellement entre 0 et 150°C dans un solvant inerte et en présence d'un accepteur d'acide tel qu'une amine tertiaire ou tel qu'un hydroxyde de métal alcalin, de préférence NaOH ou KOH. Dans le cas où l'accepteur d'acide est une amine tertiaire, on peut citer comme solvants les hydrocarbures aliphatiques ou aromatiques, éventuellement

halogénés, ou les éthers, ou les nitriles. Dans le cas où l'accepteur d'acide est un hydroxyde de métal alcalin, on peut citer comme solvants les alcools aliphatiques, tels que le méthanol, l'éthanol, l'éthylène glycol, additionnés ou non d'eau. Dans le cas où $R^4$ ou $R^6$ ou $R^{10}$ est un cation de métal alcalin ou ammonium, certains sels des produits de formule (I) sont préparés selon des procédés connus en soi, par exemple par action d'hydroxydes ou alcoolates alcalins en milieu solvant, le plus souvent simplement à température ambiante.

Les exemples suivants, donnés à titre non limitatif, illustrent l'invention et montrent comment elle peut être mise en oeuvre.

Toutes les structures des produits chimiques ont été vérifiées par spectrographie RMN (résonance magnétique nucléaire), IR (infrarouge) et SM (spectrographie de masse).

Les différentes techniques d'analyse utilisées ont montré que :

- d'une part, les produits de formule (I), dans les conditions d'analyse, existent selon les deux formes diastéréoisomères E et Z en proportions, soit équivalentes, soit prépondérantes pour l'une ou l'autre des 2 formes diastéréoisomères,
- d'autre part, les produits, dans la formule desquels il y a un groupe $R^4$ ou $R^5$ (selon la formule (I)) représentant l'atome d'hydrogène, existent dans les conditions d'analyse selon les deux formes tautomères en proportions, soit équivalentes, soit prépondérantes pour l'une ou l'autre des 2 formes tautomères.

Les exemples 1 à 31 illustrent la synthèse et les propriétés physiques de composés selon l'invention.

L'exemple 32 illustre l'application en prélevée des produits selon l'invention (les termes prélevée et pré-

émergence sont synonymes).

L'exemple 33 illustre l'application en postlevée de produits selon l'invention (les termes postlevée et post-émergence sont synonymes).

Dans ces exemples d'application, les cultures et mauvaises herbes utilisées ont été celles indiquées au tableau (II) et les résultats des exemples 32 et 33 sont rassemblés dans le tableau (III).

Exemple 1 :

Cet exemple illustre la préparation du composé N° 1 à l'aide de $PCl_5$ :

Dans 100 $cm^3$ de toluène, on met en suspension 22 g (0,05 mole) de 5-(2'-chloro-4'trifluorométhyl phénoxy) -2-nitro-N-méthanesulfonyl benzamide et 10,4 g (0,05 mole) de $PCl_5$.

On porte à l'ébullition à reflux sous agitation et poursuit le chauffage jusqu'à la fin du dégagement gazeux d'HCl (1/2 h environ). Le toluène et $POCl_3$ sont éliminés par évaporation, laissant 22,8 g d'une huile résiduelle visqueuse jaune que l'on purifie par chromatographie sur silice avec le toluène comme éluant. On obtient ainsi 18,5 g (rendement 81 %) d'un solide blanc constitué de chlorure de 5-(2'-chloro-4'-trifluorométhyl phénoxy) -2-nitro-N-méthanesulfonyl benzimidoyle fondant à 83°C et ayant pour formule :

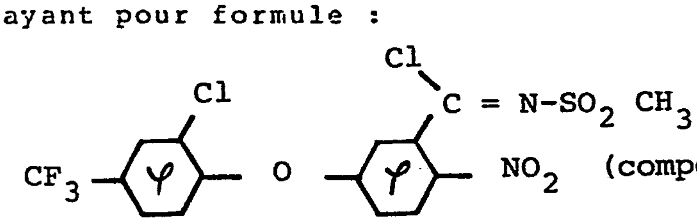

(composé n° 1)

La formule de ce produit est confirmée par spectrographie RMN et IR : bande d'absorption IR à 1650$cm^{-1}$ (C = N).

En spectrographie de masse, on observe des fragments de masse 410, 437 et 326.

Exemple 1 bis :

Cet exemple illustre la préparation du composé N° 1 à l'aide de phosgène dans 400 $cm^3$ de toluène, on met en suspension 43,9 g (0,1 mole) de 5-(2'-chloro-4' trifluorométhyl phénoxy) -2-nitro-N-méthanesulfonylbenzamide.

On agite puis on ajoute lentement, à température ambiante, 15,1 g (0,15 mole) de triéthylamine. On refroidit alors le mélange réactionnel à -10°C et le maintient à cette température tout en faisant barboter bulle à bulle 11,9 g (0,12 mole) de $COCl_2$. On poursuit l'agitation à -10°C jusqu'à la fin du dégagement gazeux de $CO_2$ (1 heure environ). On filtre le précipité de chlorhydrate de triéthylamine et lave au toluène ce précipité. Les filtrats sont réunis et le toluène est éliminé par évaporation. On obtient ainsi 43,7 g (rendement 96 %) de composé n° 1, c'est-à-dire de chlorure de 5-(2'-chloro-4'-trifluorométhyl phénoxy)-2-nitro-N-méthanesulfonyl benzimidoyle précédemment cité (composé n° 1).

Exemple 2 :

Cet exemple, dans ses variantes 2a et 2b, illustre la préparation du composé 2 à l'aide de deux réactions utilisant le méthanol, l'une comme solvant en présence de méthylate de sodium comme réactif, l'autre à la fois comme solvant et réactif.

Exemple 2a :

On prépare une solution contenant 30 g (0,066 mole) du chlorure de benzimidoyle précédent (composé n° 1) et 70 $cm^3$ de méthanol. On agite puis on ajoute lentement, à température ambiante, une solution de 3,6 g (0,066 mole) de méthylate de sodium dans 30 $cm^3$ de méthanol. On poursuit l'agitation 1 heure à température ambiante. On filtre le précipité de NaCl et le méthanol est éliminé par évaporation. L'huile résiduelle est purifiée par chromatographie sur silice avec le dichlorométhane comme éluant. On obtient ainsi 13 g (rendement 44 %) d'un solide blanc constitué de

5-(2'-chloro-4'-trifluorométhyl phénoxy) -2-nitro-N-méthanesulfonyl benzimidate de méthyle fondant à 126°C et ayant pour formule :

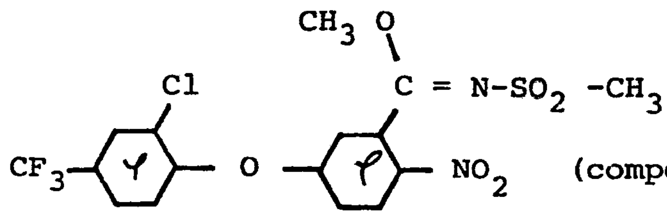

(composé n° 2)

Exemple 2b : On prépare une solution contenant 95,4 g (0,21 mole) du chlorure de benzimidoyle précédent (composé n° 1) et 200 $cm^3$ de méthanol. On agite puis on porte à l'ébullition à reflux et poursuit le chauffage jusqu'à la fin du dégagement gazeux d'HCl (1/2 heure environ). On refroidit à 5°C, puis on filtre le précipité formé et lave à l'eau ce précipité avant de le recristalliser dans le méthanol. On obtient ainsi 61,9 g (rendement 65 %) de 5-(2'-chloro-4'- trifluorométhyl phénoxy)-2-nitro-N-méthanesulfonyl benzimidate de méthyle précédemment cité.

Exemples 3 à 8 :

On prépare les différents composés 3 à 8 selon des procédés analogues à ceux de l'exemple 2.

Exemple 9 à 14 :

On synthétise les différents composés 9 à 14 selon le procédé général suivant :

On prépare une solution contenant 0,01 mole de composé de formule $R^3OH$, 1,4 $cm^3$ de triéthylamine et 10$cm^3$ d'éther éthylique. On agite, refroidit à 5°C, et ajoute goutte à goutte une solution de 4,6 g de chlorure de 5-(2'-chloro-4'-trifluorométhyl phénoxy) -2-nitro-N-méthanesulfonyl benzimidoyle (composé n° 1) dans 20 $cm^3$ d'éther. On poursuit l'agitation pendant 4 heures à température ambiante. On filtre le précipité de chlorhydrate de triéthylamine et lave à l'éther ce précipité. Les filtrats sont réunis, lavés à l'eau, séchés sur sulfate de sodium et

concentrés ; l'huile résiduelle est purifiée par chromatographie sur silice avec le dichlorométhane comme éluant.

Exemples 15 à 18 :

On synthétise les différents composés 15 à 18 selon le procédé général suivant :

On prépare une solution contenant 0,01 mole de composé de formule $R^3OH$, 1,4 $cm^3$ de triéthylamine et 10 $cm^3$ de toluène. On agite puis on ajoute lentement, à température ambiante, une solution de 4,6 g de chlorure de 5-(2'-chloro-4'-trifluorométhyl phénoxy) -2-nitro-N-méthanesulfonyl benzimidoyle (composé n° 1) dans 20 $cm^3$ de toluène. On porte le mélange réactionnel à 90°C et poursuit le chauffage pendant 3 heures à cette température. On filtre le précipité de chlorhydrate de triéthylamine et lave au toluène ce précipité. Les filtrats sont réunis, lavés à l'eau, séchés sur sulfate de sodium et concentrés ; l'huile résiduelle est purifiée par chromatographie sur silice avec le dichlorométhane comme éluant.

Exemples 19 à 21 :

On prépare les deux composés 19 et 21 selon le procédé général des exemples 9 à 14, mais en remplaçant le composé de formule $R^3OH$ par un composé de formule $R^4SH$. Le composé 20, quant à lui, est synthétisé par réaction, à température ambiante, du composé 19 constitué de 5-(2'-chloro-4'-trifluorométhyl phénoxy) -2-nitro-N-méthanesulfonyl thiobenzamide avec une quantité stoechiométrique de soude aqueuse (ou de méthylate de sodium en solution dans le méthanol), suivie de l'élimination par évaporation de l'eau (ou du méthanol), selon un procédé connu en soi.

Exemples 22 et 23 :

On prépare les deux composés 22 et 23 selon le procédé général des exemples 15 à 18, mais en remplaçant le composé de formule $R^3OH$ par un composé de formule $R^4SH$.

Exemples 24 à 27 :

On prépare les différents composés 24 à 27 selon le procédé général des exemples 9 à 14, mais en remplaçant le composé de formule $R^3OH$ par un composé de formule $R^5R^6NH$.

Exemple 28 :

On prépare une solution contenant 3 g (0,0066 mole) du composé 27 constitué de 5-(2'-chloro-4'-trifluorométhyl phénoxy)-2-nitro-N-méthanesulfonyl-N'-hydroxy benzamidine, ainsi que 20 $cm^3$ d'éthanol et 5 $cm^3$ d'eau. On agite puis on ajoute successivement, à température ambiante, 1,1 g (0,0066 mole) de 2-bromopropionate de méthyle et 0,26 g (0,0066 mole) de soude. On porte à l'ébullition à reflux et poursuit le chauffage pendant 2 heures. L'éthanol et l'eau sont éliminés par évaporation ; le résidu est repris au dichlorométhane. La phase organique est lavée à l'eau, séchée sur sulfate de sodium et concentrée ; l'huile résiduelle est purifiée par chromatographie sur silice avec le mélange dichlorométhane/méthanol 97/3 comme éluant. On obtient ainsi 1,2 g (rendement 34 %) d'une masse visqueuse constituée de 5-(2'-chloro-4'-trifluorométhyl phénoxy) -2-nitro-N-méthanesulfonyl-N'-(1-carbométhoxy)éthoxy benzamidine ayant pour formule :

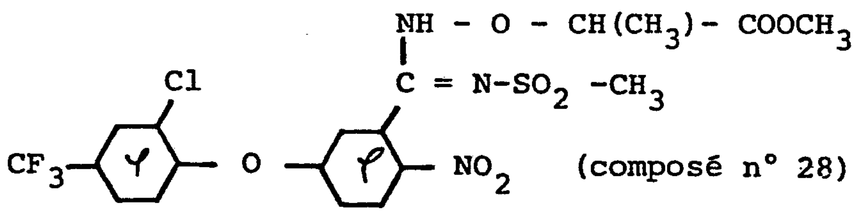

(composé n° 28)

Exemples 29 et 30 :

On prépare une suspension de 1,3 g (0,01 mole) de sel de potassium du méthanesulfonamide dans 30 $cm^3$ de toluène. On agite puis on ajoute, à température ambiante, une solution de 4,6 g (0,01 mole) de chlorure de

5-(2'-chloro-4'-trifluorométhyl phénoxy) -2-nitro-N-méthanesulfonyl benzimidoyle (composé n° 1) dans 20 $cm^3$ de toluène. On porte à l'ébullition à reflux et poursuit le chauffage pendant 4 heures. On filtre le précipité de KCl. Le filtrat organique est lavé à l'eau, séché sur sulfate de sodium et concentré ; l'huile résiduelle est purifiée par chromatographie sur silice avec le mélange dichlorométhane/méthanol 90/10 comme éluant. On obtient ainsi 1,5 g (rendement 29 %) d'un solide blanc constitué de 5-(2'-chloro-4'-trifluorométhyl phénoxy) -2-nitro-N,N'-bis-méthanesulfonyl benzamidine fondant à 211°C et ayant pour formule :

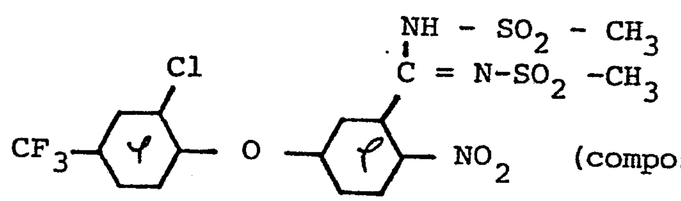

(composé n° 29)

Le composé 30, quant à lui, est synthétisé par réaction, à température ambiante, du composé précédent avec une quantité stoechiométrique de soude aqueuse (ou de méthylate de sodium en solution dans le méthanol), suivie de l'élimination par évaporation de l'eau (ou du méthanol), selon un procédé connu en soi.

Les différents composés obtenus dans les divers exemples 1 à 30 (un composé a le numéro de l'exemple qui lui correspond) ont pour formule :

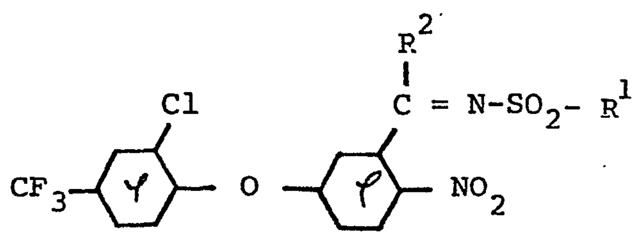

La signification des substituants $R^1$ et $R^2$ pour les différents composés ainsi que le rendement de l'exemple

et le point de fusion du produit sont rassemblés au tableau (I).

Pour le composé n° 19, la seconde forme tautomère de formule :

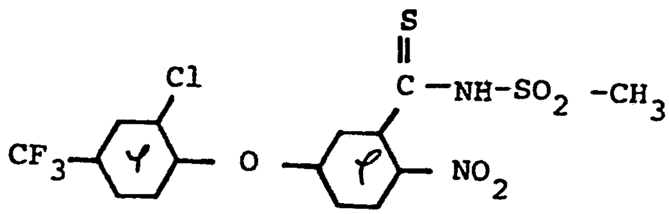

est prépondérante.

Les composés comme le n° 15, ou le n° 16, ou le n° 23, ou le n° 28, qui présentent un carbone asymétrique, existent selon les deux formes énantiomères R ou S. Dans ce cas, la formule (I) donnée ci-avant doit bien être comprise comme définissant l'une ou l'autre de ces deux formes, ou comme définissant le mélange des deux en proportions, soit équivalentes (racémate), soit prépondérantes pour l'une ou l'autre d'entre elles.

<u>Exemple 31</u> :

Dans 50 $cm^3$ de toluène, on met en suspension 7,4 g (0,018 mole) de 5-(2,4-dichloro-phénoxy)-2-nitro-N-méthanesulfonyl benzamide et 3,8 g (0,018 mole) de $PCl_5$.

On porte à l'ébullition à reflux sous agitation et poursuit le chauffage jusqu'à la fin du dégagement gazeux d'HCl (1/2 heure environ). Le toluène et $POCl_3$ sont éliminés par évaporation, laissant 7,7 g d'une huile résiduelle visqueuse brune, que l'on dissout dans 25 $cm^3$ de méthanol. On agite cette solution méthanolique puis on porte à l'ébullition à reflux et poursuit le chauffage jusqu'à la fin du dégagement gazeux d'HCl (1/2 heure environ). On refroidit à 5°C, puis on filtre le précipité formé avant de le recristalliser dans le méthanol. On obtient

ainsi 4,6 g (rendement 60 %) d'une solide blanc de 5-(2',4'-dichlorophénoxy)-2-nitro-N-méthanesulfonyl benzimidate de méthyle fondant à 163°C et ayant pour formule :

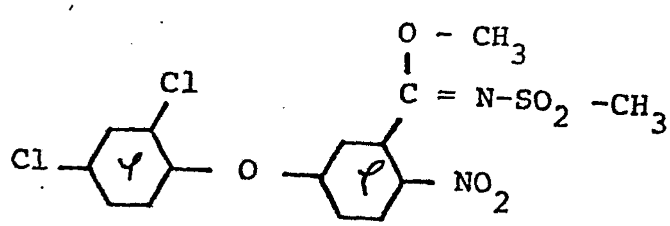

Les composés suivants peuvent être préparés selon un procédé analogue à celui de l'exemple 31 :

. 5-(2'-chloro-4'-trifluorométhyl phénoxy)-2-chloro-N-méthanesulfonyl benzimidate de méthyle,

. 5-(2'-chloro-4'-trifluorométhyl phénoxy)-2-bromo-N-méthanesulfonyl benzimidate de méthyle,

. 5-(2'-chloro-4'-trifluorométhyl phénoxy)-2-cyano-N-méthanesulfonyl benzimidate de méthyle,

. 5-(2',4'-dichlorophénoxy)-2-chloro-N-méthanesulfonyl benzimidate de méthyle,

. 5-(2',4'-dichlorophénoxy)-2-bromo-N-méthanesulfonyl benzimidate de méthyle,

. 5-(2',4'-dichlorophénoxy)-2-cyano-N-méthanesulfonyl benzimidate de méthyle,

. 5-(2',6'-dichloro-4'-trifluorométhyl phénoxy)-2-chloro-N-méthanesulfonyl benzimidate de méthyle,

. 5-(2'-bromo-4'-trifluorométhyl phénoxy)-2-nitro-N-méthanesulfonyl benzimidate de méthyle,

. 5-(2'-iodo-4'-trifluorométhyl phénoxy)-2-nitro-N-méthanesulfonyl benzimidate de méthyle,

. 5-(2'-nitro-4'-trifluorométhyl phénoxy)-2-nitro-N-méthanesulfonyl benzimidate de méthyle,

. 5-(2'-cyano-4'-trifluorométhyl phénoxy)-2-nitro-N-méthanesulfonyl benzimidate de méthyle,

. 5-(bis-2',4'-trifluorométhyl phénoxy)-2-nitro-N-méthanesulfonyl benzimidate de méthyle,

. 5-(2',6'-dichloro-4'-trifluorométhyl phénoxy)-2-nitro-N-méthanesulfonyl benzimidate de méthyle,

. 5-(2'-chloro-6'-fluoro-4'-trifluorométhyl phénoxy)-2-nitro-N-méthanesulfonyl benzimidate de méthyle,

. 5-(2',3',6'-trichloro-4'-trifluorométhyl phénoxy)-2-nitro-N-méthanesulfonyl benzimidate de méthyle,

. 5-(2'-nitro-6'-chloro-4'-trifluorométhyl phénoxy)-2-nitro-N-méthanesulfonyl benzimidate de méthyle,

. 5-(2',3',5',6'-tétrafluoro-4'-trifluorométhyl phénoxy)-2-nitro-N-méthanesulfonyl benzimidate de méthyle,

. 5-(2'-trifluorométhyl-4'-chlorophénoxy) -2-nitro-N-méthanesulfonyl benzimidate de méthyle,

. 5-(2'-méthyl-4'-chlorophénoxy)-2-nitro-N-méthanesulfonyl benzimidate de méthyle,

. 5-(2'-nitro-4'-chlorophénoxy)-2-nitro-N-méthanesulfonyl benzimidate de méthyle,

. 5-(2',4',6'-trichlorophénoxy)-2-nitro-N-méthanesulfonyl benzimidate de méthyle,

.5-(2',4'-dichloro-6'-fluorophénoxy)-2-nitro-N-méthanesulfony l benzimidate de méthyle,

. 5-(2',3',4'-trichlorophénoxy)-2-nitro-N-méthanesulfonyl benzimidate de méthyle,

. 5-(2',4',5'-trichlorophénoxy)-2-nitro-N-méthanesulfonyl benzimidate de méthyle,

. 5-(2',3',4',6'-tétrachlorophénoxy) -2-nitro-N-méthanesulfonyl benzimidate de méthyle,

. 5-(2'-chloro-4'-bromophénoxy)-2-nitro-N-méthanesulfonyl benzimidate de méthyle,

. 5-(2',4'-dibromophénoxy)-2-nitro-N-méthanesulfonyl benzimidate de méthyle,

. 5-(2',4',6'-tribromophénoxy)-2-nitro-N-méthanesulfonyl benzimidate de méthyle,

. 5-(2'-chloro-4'-fluorophénoxy)-2-nitro-N-méthanesulfonyl benzimidate de méthyle,

. 5-(2'-chloro-4'-méthylphénoxy)-2-nitro-N-méthanesulfonyl benzimidate de méthyle

. 5-(2'-nitro-4'-méthylphénoxy)-2-nitro-N-méthanesulfonyl benzimidate de méthyle

. 5-(2',3',6'-trichloro-4'-méthylphénoxy)-2-nitro-N-méthanesulfonyl benzimidate de méthyle

. 5-(2'-chloro-4'-éthylphénoxy)-2-nitro-N-méthanesulfonyl benzimidate de méthyle,

. 5-(2',6'-dichloro-4'-éthylphénoxy)-2-nitro-N-méthanesulfonyl benzimidate de méthyle,

. 5-(2',3',5',6'-tétrafluoro-4'-éthylphénoxy)-2-nitro-N-méthanesulfonyl benzimidate de méthyle,

. 5-(3',5'-dichlorophénoxy)-2-nitro-N-méthanesulfonyl benzimidate de méthyle,

. 5-(2'-chloro-4'-trifluorométhyl phénoxy)-2-nitro-N-n-propanesulfonyl benzimidate de méthyle,

. 5-(2'-chloro-4'-trifluorométhyl phénoxy)-2-nitro-N-trifluorométhanesulfonyl benzimidate de méthyle,

. 5-(2'-chloro-4'-trifluorométhyl phénoxy)-2-nitro-N-vinylsulfonyl benzimidate de méthyle,

. 5-(2'-chloro-4'-trifluorométhyl phénoxy)-2-nitro-N-éthynylsulfonyl benzimidate de méthyle,

. 5-(2'-chloro-4'-trifluorométhyl phénoxy)-2-nitro-N-allylsulfonyl benzimidate de méthyle,

. 5-(2'-chloro-4'-trifluorométhyl phénoxy)-2-nitro-N-propargylsulfonyl benzimidate de méthyle.

Exemple 32 :

Application herbicide, en prélevée des expèces végétales -

Dans des pots de 9 x 9 x 9 cm remplis de terre agricole légère, on sème un nombre de graines déterminé en fonction de l'espèce végétale et de la grosseur de la graine.

Les pots sont traités par pulvérisation de bouillie en quantité correspondant à une dose volumique d'application de 500 l/ha et contenant la matière active à la concentration désirée.

Le traitement avec la bouillie est donc effectué sur des graines non recouvertes de terre (on utilise le terme de bouillie pour désigner, en général, les compositions diluées à l'eau, telles qu'on les applique sur les végétaux).

La bouillie utilisée pour le traitement est une suspension aqueuse de la matière active contenant 0,1 % en poids de Cémulsol NP 10 (agent tensio-actif constitué d'alkylphénol polyéthoxylé, notamment de nonylphénolpolyéthoxylé) et 0,04 % en poids de tween 20 (agent tensio-actif constitué d'un oléate de dérivé polyoxyéthylèné du sorbitol).

Cette suspension a été obtenue en mélangeant et broyant les ingrédients dans un microniseur, de façon à obtenir une grosseur moyenne de particules inférieures à 40 microns.

Selon la concentration en matière active de la bouillie, la dose de matière active appliquée a été de 0,125 à 4 kg/ha.

Après traitement, on recouvre les graines d'une couche de terre d'environ 3 mm d'épaisseur.

Les pots sont alors placés dans des bacs destinés à recevoir l'eau d'arrosage, en subirrigation, et maintenus pendant 21 jours à température ambiante sous 70 % d'humidité relative.

Au bout de 21 jours, on compte le nombre de plantes vivantes dans les pots traités par la bouillie contenant la matière active à tester et le nombre de plantes vivantes dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100 % indique qu'il y a eu destruction complète de l'espèce végétale considérée et un pourcentage de 0 % indique que le nombre de plantes vivantes dans le pot traité est identique à celui dans le pot témoin.

Exemple 33 :

Application herbicide, en postlevée des espèces végétales -

Dans des pots de 9 x 9 x 9 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur et on laisse germer la graine jusqu'à ce qu'elle donne naissance à une plantule au stade convenable. Le stade de traitement pour les graminées est le stade "deuxième feuille en formation". Le stade convenable pour le soja est le stade "première feuille trifoliée étalée". Le stade de traitement pour les autres dicotylédones, est le stade "cotylédons étalés, première feuille vraie en développement".

Les pots sont alors traités par pulvérisation de bouillie en quantité correspondant à une dose volumique d'application de 500 l/ha et contenant la matière active à la concentration désirée.

La bouille a été préparée de la même manière qu'à l'exemple 32.

Selon la concentration en matière active de la bouillie, la dose de matière active appliquée a été de 0,125 à 4 kg/ha.

Les pots traités sont ensuite placés dans des bacs destinés à recevoir l'eau d'arrosage, en subirrigation, et maintenus pendant 21 jours à température ambiante sous 70 % d'humidité relative.

Au bout de 21 jours, on compte le nombre de plantes vivantes dans les pots traités par la bouillie contenant la matière active à tester et le nombre de plantes vivantes

dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100 % indique qu'il y a eu destruction complète de l'espèce végétale considérée et un pourcentage de 0 % indique que le nombre de plantes vivantes dans le pot traité est identique à celui dans le pot témoin.

Les essais réalisés montrent donc les propriétés remarquablement avantageuses des composés selon l'invention, aussi bien pour des traitements en pré-levée des cultures, que pour le traitement en post-levée, notamment du soja, des céréales dont le maïs, du tournesol, du cotonnier et du haricot. Dans le cas du soja, l'activité des composés est particulièrement intéressante lorque cette culture est infestée de mauvaises herbes dicotylédones telles que l'abutilon, le Xanthium et l'Ipomea.

Pour leur emploi pratique, les composés selon l'invention sont rarement utilisés seuls. Le plus souvent ces composés font partie de compositions. Ces compositions, utilisables comme agents herbicides, contiennent comme matière active un composé selon l'invention tel que décrit précédemment en association avec les supports solides ou liquides, acceptables en agriculture et les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants,

des séquestrants, etc... ainsi que d'autres matières actives connues à propriétés pesticides (notamment insecticides, fongicides ou herbicides) ou à propriétés favorisant la croissance des plantes (notamment des engrais) ou à propriétés régulatrices de la croissance des plantes. Plus généralement les composés utilisés dans l'invention peuvent être associés à tous les additifs solides ou liquides correspondant aux techniques habituelles de la mise en formulation.

Les doses d'emploi des composés utilisés dans l'invention peuvent varier dans de larges limites, notamment selon la nature des adventices à éliminer et le degré d'infestation habituel des cultures pour ces adventices.

D'une façon générale, les compositions selon l'invention contiennent habituellement de 0,05 à 95 % environ (en poids) d'une ou plusieurs matières actives selon l'invention, de 1 % à 95 % environ de un ou plusieurs supports solides ou liquides et éventuellement de 0,1 à 20% environ de un ou plusieurs agents tensioactifs.

Selon ce qui a déjà été dit les composés utilisés dans l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol ; esters, notamment l'acétate de méthyl-glycol ; cétones, notamment la cyclohexanone et l'isophorone ; fractions de pétrole ; hydrocarbures aromatiques, notamment les xylènes, ou paraffiniques ; hydrocarbures chlorés aliphatiques, notamment le trichloroéthane,

ou aromatiques, notamment les chlorobenzènes ; des solvants hydrosolubles tels que le diméthylformamide, le diméthylsulfoxyde, la N-méthyl-pyrrolidone ; gaz liquéfiés, etc..).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 0,5 et 80 % pour ces derniers cas).

Comme formes de compositions liquides ou destinées à constituer des compositions liquides lors de l'application, on peut citer les solutions, en particulier les concentrés émulsionnables, les émulsions, les suspensions concentrées, les aérosols, les poudres mouillables (ou poudre à pulvériser), les granulés autodispersibles et les pâtes.

Les concentrés émulsionnables ou solubles comprennent également le plus souvent 10 à 80 % de matière active, les émulsions ou solutions prêtes à l'application contenant, quant à elles, 0,01 à 20 % de matière active. En plus du solvant, les concentrés émulsionnables peuvent contenir, quand c'est nécessaire, 2 à 20 % d'additifs appropriés, comme des stabilisants, des agents tensioactifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les végétaux.

A titre d'exemple, voici la composition de quelques concentrés émulsionnables :

| | |
|---|---|
| - matière active | 250 g |
| - alkylphénol polyéthoxylé | 30 g |
| - alkylarylsulfonate de calcium | 50 g |
| - coupe de distillation du pétrole, distillant entre 160 et 185°C | 670 g |

Autre formule :

| | |
|---|---|
| - matière active | 350 g |
| - huile de ricin polyéthoxylée | 60 g |
| - alkylarylsulfonate de sodium | 40 g |
| - cyclohexanone | 150 g |
| - xylène | 400 g |

Autre formule :

| | |
|---|---|
| - matière active | 400 g |
| - alkyl phénol polyéthoxylé | 100 g |
| - éther méthylique de l'éthylène glycol | 250 g |
| - coupe pétrolière aromatique distillant entre 160-185°C | 250 g |

Autre formule :

| | |
|---|---|
| - matière active | 400 g |
| - phosphate de tristyrylphénol polyéthoxylé | 50 g |
| - phosphate d'alkylphénol polyéthoxylé | 65 g |
| - alkyl benzène sulfonate de sodium | 35 g |
| - cyclohexanone | 300 g |
| - coupe pétrolière aromatique distillant entre 160-185°C | 150 g |

Autre formule :

| | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique | q.s.p. 1 litre. |

Selon une autre formule de concentré émulsionnable, on utilise :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

Les suspensions concentrées, qui sont applicables en pulvérisation, sont préparées de manière à obtenir un produit fluide stable ne se déposant pas (broyage fin) et elles contiennent habituellement de 10 à 75 % de matière active, de 0,5 à 15 % d'agents tensioactifs, de 0,1 à 10 % d'agents thixotropes, de 0 à 10 % d'additifs appropriés,

comme des anti-mousses, des inhibiteurs de corrosion, des stabilisants, des agents de pénétration et des adhésifs et, comme support, de l'eau ou un liquide organique dans lequel la matière active est peu soluble ou non soluble : certaines matières solides organiques ou des sels minéraux peuvent être dissous dans le support pour aider à empêcher la sédimentation ou comme antigels pour l'eau.

A titre d'exemple, voici une composition de suspension concentrée :

| | |
|---|---|
| - matière active | 500 g |
| - phosphate de tristyrylphénol polyéthoxylé | 50 g |
| - alkylphénol polyéthoxylé | 50 g |
| - polycarboxylate de sodium | 20 g |
| - éthylène glycol | 50 g |
| - huile organopolysiloxanique (antimousse) | 1 g |
| - polysaccharide | 1,5 g |
| - eau | 316,5 g |

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quant c'est nécessaire, de 0 à 10 % d'un ou plusieurs stabilisants et/ou autres additifs, comme des agents de pénétration, des adhésifs, ou des agents antimottants, colorants, etc..

A titre d'exemple, voici diverses compositions de poudres mouillables :

| | |
|---|---|
| - matière active | 50 % |
| - lignosulfonate de calcium (défloculant) | 5 % |
| - isopropylnaphtalène sulfonate (agent mouillant anionique) | 1 % |
| - silice antimottante | 5 % |
| - kaolin (charge) | 39 % |

Un autre exemple de poudre mouillable à 80 % est donné ci-après :

| | |
|---|---|
| - matière active | 80 % |
| - alkylnaphtalène sulfonate de sodium | 2 % |
| - lignosulfonate de sodium | 2 % |
| - silice antimottante | 3 % |
| - kaolin | 13 % |

Un autre exemple de poudre mouillable est donné ci-après :

| | |
|---|---|
| - matière active | 50 % |
| - alkylnaphtalène sulfonate de sodium | 2 % |
| - méthyl cellulose de faible viscosité | 2 % |
| - terre de diatomées | 46 % |

Un autre exemple de poudre mouillable est donné ci-après :

| | |
|---|---|
| - matière active | 90 % |
| - dioctylsulfosuccinate de sodium | 0,2 % |
| - silice synthétique | 9,8 % |

Une autre composition de poudre à pulvériser à 40 % utilise les constituants suivants :

| | |
|---|---|
| - matière active | 400 g |
| - lignosulfonate de sodium | 50 g |
| - dibutylnaphtalène sulfonate de sodium | 10 g |
| - silice | 540 g |

Une autre composition de poudre à pulvériser à 25 % utilise les constituants suivants :

| | |
|---|---|
| - matière active | 250 g |
| - isooctylphénoxy-polyoxyéthylène-éthanol | 25 g |
| - mélange équipondéral de craie de Champagne et d'hydroxyéthylcellulose | 17 g |
| - aluminosilicate de sodium | 543 g |
| - kieselguhr | 165 g |

Une autre composition de poudre à pulvériser à 10 % utilise les constituants suivants :

| | |
|---|---|
| - matière active | 100 g |
| - mélange de sels de sodium de sulfates d'acides gras saturés | 30 g |
| - produit de condensation d'acide naphtalène sulfonique et de formaldéhyde | 50 g |
| - kaolin | 820 g |

Pour obtenir ces poudres à pulvériser ou poudres mouillables, on mélange intimement les matières actives dans des mélangeurs appropriés avec les substances additionnelles ou on imprègne la matière active fondue sur la charge poreuse et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles de végétaux.

Les granulés "autodispersibles" (en langue anglaise "dry flowable" ; il s'agit plus exactement de granulés facilement dispersibles dans l'eau) ont une composition sensiblement voisine de celle des poudres mouillables. Ils peuvent être préparés par granulation de formulations décrites pour les poudres mouillables, soit par voie humide (mise en contact de la matière active finement divisée avec la charge inerte et avec un peu d'eau, par exemple 1 à 20%, ou de solution aqueuse de dispersant ou de liant, puis séchage et tamisage), soit par voie sèche (compactage puis broyage et tamisage).

A titre d'exemple, voici une formulation de granulé autodispersible :

| | |
|---|---|
| - matière active | 800 g |
| - alkylnaphtalène sulfonate de sodium | 20 g |
| - méthylène bis naphtalène sulfonate de sodium | 80 g |
| - kaolin | 100 g |

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaisse comme celle d'une "mayonnaise".

Toutes ces dispersions ou émulsions aqueuses ou bouillies sont applicables aux cultures à désherber par tout moyen convenable, principalement par pulvérisation, à des doses qui sont généralement de l'ordre de 100 à 1 200 litres de bouillies à l'hectare.

Les granulés destinés à être disposés sur le sol sont habituellement préparés de manière qu'ils aient des dimensions comprises entre 0,1 et 2 mm et ils peuvent être fabriqués par agglomération ou imprégnation. De préférence, les granulés contiennent 1 à 25 % de matière active et 0 à 10 % d'additifs comme des stabilisants, des agents de modification à libération lente, des liants et des solvants.

Selon une exemple de composition de granulé, on utilise les constituants suivants :

- matière active 50 g
- propylène glycol 25 g
- huile de lin bouillie 50 g
- argile (granulométrie : 0,3 à 0,8 mm) 910 g

Comme indiqué plus haut, l'invention concerne également un procédé de désherbage de cultures, notamment les céréales telles que le blé, l'orge et le maïs, ainsi que le soja, le tournesol, le cotonnier et le haricot, selon lequel on applique sur les plantes et/ou sur le sol de la zone à désherber une quantité efficace, et non phytotoxique vis-à-vis de la culture considérée, d'au moins un des composés selon l'invention. Ceux-ci sont utilisés pratiquement sous forme des compositions herbicides selon l'invention qui ont été décrites ci-avant. Généralement, des quantités de matière active allant de 0,01 à 5 kg/ha, de préférence de 0,1 à 2 kg/ha, donnent de bons résultats, étant entendu que le choix de la quantité de matière active à utiliser est fonction de l'intensité du problème à résoudre, des conditions climatiques et de la culture considérée. Le traitement peut être effectué soit en prélevée des cultures et adventices, ou en présemis des cultures avec incorporation dans le sol (cette incorporation est donc une opération complémentaire du procédé de traitement de l'invention), soit en postlevée. D'autres modes de mise en oeuvre du procédé de traitement selon l'invention peuvent encore être utilisés : ainsi on peut appliquer la matière active sur le sol, avec ou sans incorporation, avant repiquage d'une culture.

Le procédé de traitement de l'invention s'applique aussi bien dans le cas de cultures annuelles que dans le cas de cultures pérennes ; dans ce dernier cas, on préfère appliquer les matières actives de l'invention de manière localisée, par exemple entre les rangs des dites cultures.

TABLEAU (I)

| Composé No. | $R^1$ | $R^2$ | Rendement en % | Point de fusion en °C |
|---|---|---|---|---|
| 1 | $CH_3$ | Cl | 96 | 83 |
| 2 | $CH_3$ | $CH_3O-$ | 65 | 126 |
| 3 | $CH_3$ | $C_2H_5O-$ | 54 | 102 |
| 4 | $CH_3$ | $(CH_3)_2CHO-$ | 37 | 125 |
| 5 | $C_2H_5$ | $CH_3O-$ | 45 | 112 |
| 6 | $ClCH_2$ | $CH_3O-$ | 54 | 127 |
| 7 | 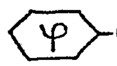 $-CH_2-$ | $CH_3O-$ | 29 | 100 |
| 8 | Cl- 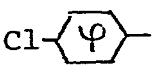 | $CH_3O-$ | 49 | produit visqueux |
| 9 | $CH_3$ | $ClCH_2CH_2O-$ | 71 | 101 |
| 10 | $CH_3$ | $CH_3OCH_2CH_2O-$ | 34 | 128 |
| 11 | $CH_3$ | $CCl_3CH_2O-$ | 67 | 177 |
| 12 | $CH_3$ | $CF_3CH_2O-$ | 92 | 144 |
| 13 | $CH_3$ | $CH_2=CHCH_2O-$ | 46 | 88 |
| 14 | $CH_3$ | $HC\equiv C-CH_2O-$ | 67 | 134 |
| 15 | $CH_3$ | $C_2H_5O_2C\underset{CH_3}{C}HO-$ | 60 | produit visqueux |
| 16 | $CH_3$ | 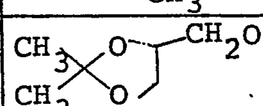 | 73 | 68 |
| 17 | $CH_3$ | $(CH_3)_2C=NO-$ | 91 | produit visqueux |
| 18 | $CH_3$ | 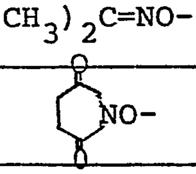 | 39 | 95 |

TABLEAU (I) (Suite)

| Composé No. | $R^1$ | $R^2$ | Rendement en % | Point de fusion en °C |
|---|---|---|---|---|
| 19 | $CH_3$ | HS- | 44 | 182 |
| 20 | $CH_3$ | NaS- | 93 | 220 |
| 21 | $CH_3$ | $CH_3S$- | 75 | 47 |
| 22 | $CH_3$ | $C_2H_5$-O-OC-$CH_2$-S- | 70 | 91 |
| 23 | $CH_3$ | $C_2H_5$-O-OC-CH($CH_3$)-S- | 98 | produit visqueux |
| 24 | $CH_3$ | $H_2N$ | 80 | 79 |
| 25 | $CH_3$ | ($CH_3$)HN- | 76 | 75 |
| 26 | $CH_3$ | $(CH_3)_2N$- | 61 | 69 |
| 27 | $CH_3$ | (HO)HN- | 100 | 150 |
| 28 | $CH_3$ | $CH_3$-O-OC-CH($CH_3$)-O-NH- | 34 | produit visqueux |
| 29 | $CH_3$ | $CH_3SO_2$-NH- | 29 | 211 |
| 30 | $CH_3$ | $(CH_3SO_2)$NaN- | 100 | 110 |

TABLEAU II

| | Nom Français | Nom Américain | Nom Latin | Abréviation |
|---|---|---|---|---|
| Culture | Blé | Wheat | | BLE |
| | Soja | Soybean | | SOJ |
| Mauvaises herbes | Panisse | Barnyardgrass | Echinochloa crus-galli | ECH |
| | | Velvet leaf | Abutilon theophrasti | ABU |
| | | Cocklebur | Xanthium pennsylvanicum | XAN |
| | Moutarde sauvage | Wild mustard | Sinapis arvensis | SIN |
| | Liseron pourpre | Morningglory (annual) | Ipomea purpurea | IPO |
| | Ray-grass | Rye-grass | Lolium multiflorum | LOL |

# TABLEAU (III)

| N°Composé | Doses en kg/ha | PRE-LEVEE | | | | | | | | POST-LEVEE | | | | | | | | |
|---|---|---|---|---|---|---|---|---|---|---|---|---|---|---|---|---|---|---|
| | | ECH | LOL | ABU | IPO | SIN | XAN | BLE | SOJ | ECH | LOL | ABU | IPO | SIN | XAN | CYP | BLE | SOJ |
| 1 | 4 | | | | | | | | | | | | | | | | | |
| | 1 | 100 | 100 | 100 | 100 | 100 | 100 | 100 | 0 | 100 | 30 | 100 | 80 | 100 | 100 | 50 | 50 | 0 |
| | 0,25 | 100 | 50 | 100 | 80 | 100 | 60 | 20 | 0 | 100 | 0 | 50 | 80 | 100 | 100 | 0 | 0 | 0 |
| 2 | 4 | 100 | 100 | 100 | 30 | 100 | 0 | 90 | 80 | 100 | 100 | 100 | 100 | 100 | 100 | 0 | 20 | 30 |
| | 1 | 100 | 100 | 100 | 0 | 98 | 0 | 60 | 100 | 60 | 80 | 100 | 100 | 100 | 100 | 0 | 0 | 0 |
| | 0,25 | 100 | 90 | 100 | 0 | 98 | 0 | 60 | 0 | 50 | 40 | 80 | 100 | 100 | 100 | 0 | 0 | 0 |
| 3 | 4 | 95 | 98 | 100 | 0 | 50 | 0 | 20 | 0 | 80 | 50 | 100 | 100 | 70 | 90 | 0 | 0 | 0 |
| | 1 | 60 | 80 | 100 | 0 | 0 | 0 | 0 | 0 | 30 | 0 | 20 | 100 | 30 | 90 | 0 | 0 | 0 |
| | 0,25 | 50 | 50 | 100 | 0 | 0 | 0 | 0 | 0 | 20 | 0 | 0 | 90 | 30 | 80 | 0 | 0 | 0 |
| 4 | 4 | 40 | 0 | 100 | 0 | 0 | 0 | 0 | 0 | 20 | 20 | 0 | 50 | 60 | 100 | 0 | 0 | 0 |
| | 1 | 0 | 0 | 100 | 0 | 0 | 0 | 0 | 0 | 0 | 20 | 0 | 0 | 50 | 80 | 0 | 0 | 0 |
| | 0,25 | 0 | 0 | 100 | 0 | 0 | 0 | 0 | 0 | 0 | 20 | 0 | 0 | 50 | 100 | 0 | 0 | 0 |

TABLEAU (III) (Suite)

| N°Composé | Doses en kg/ha | PRE-LEVEE | | | | | | | | POST-LEVEE | | | | | | | | |
|---|---|---|---|---|---|---|---|---|---|---|---|---|---|---|---|---|---|---|
| | | ECH | LOL | ABU | IPO | SIN | XAN | BLE | SOJ | ECH | LOL | ABU | IPO | SIN | XAN | | BLE | SOJ |
| 5 | 2 | 80 | 90 | 100 | 80 | 80 | 90 | 30 | 0 | 20 | 30 | 80 | 90 | 90 | | | 10 | 0 |
| | 0,5 | 60 | 80 | 100 | 30 | 90 | | 0 | | 10 | 20 | 80 | 100 | 90 | | | 0 | 0 |
| | 0,125 | 20 | 40 | 90 | 50 | 20 | | 0 | 0 | 10 | 10 | 10 | 10 | 70 | | | 0 | 0 |
| 6 | 2 | 50 | 50 | 80 | 90 | 80 | | 0 | | 20 | 20 | 20 | 70 | 100 | | | 0 | 0 |
| | 0,5 | 40 | 40 | 90 | 60 | 50 | | 0 | | 10 | 10 | 10 | 40 | 90 | | | 0 | 0 |
| | 0,125 | 0 | 0 | 90 | 50 | 0 | | 0 | | 0 | 0 | 0 | 0 | 60 | | | 0 | 0 |
| 7 | 2 | 0 | 0 | 100 | 0 | 20 | | 0 | 0 | 40 | 0 | 80 | 100 | 100 | | | 0 | 0 |
| | 0,5 | 0 | 0 | 20 | 0 | 0 | | 0 | 0 | 20 | 0 | 80 | 90 | 50 | | | 0 | 0 |
| | 0,125 | 0 | 0 | 0 | 0 | 0 | | 0 | 0 | 0 | 0 | 20 | 80 | 0 | | | 0 | 0 |
| 8 | 2 | 30 | 10 | 20 | 30 | 100 | | 0 | 0 | 50 | 20 | 70 | 90 | 100 | | | 0 | 0 |
| | 0,5 | 0 | 10 | 20 | 20 | 0 | | 0 | 0 | 20 | 0 | 50 | 70 | 90 | | | 0 | 0 |
| | 0,125 | 0 | 0 | 10 | 0 | 0 | | 0 | 0 | 10 | 0 | 20 | 0 | 10 | | | 0 | 0 |

TABLEAU (III) (suite)

| N°Composé | Doses en kg/ha | PRE-LEVEE | | | | | | | | POST-LEVEE | | | | | | | | |
|---|---|---|---|---|---|---|---|---|---|---|---|---|---|---|---|---|---|---|
| | | ECH | LOL | ABU | IPO | SIN | XAN | BLE | SOJ | ECH | LOL | ABU | IPO | SIN | XAN | | BLE | SOJ |
| 9 | 4 | 100 | 98 | 100 | 90 | 95 | | 0 | 0 | 40 | 80 | 50 | 100 | 100 | | | 0 | 0 |
| | 1 | 50 | 60 | 98 | 0 | 95 | | 0 | 0 | 20 | 50 | 30 | 80 | 95 | | | 0 | 0 |
| | 0,25 | 0 | 0 | 50 | 0 | 0 | | 0 | 0 | 0 | 0 | 0 | 100 | 30 | | | 0 | 0 |
| 10 | 2 | 100 | 100 | 100 | 100 | 100 | | 20 | 0 | 100 | 60 | 80 | 100 | 100 | | | 20 | 0 |
| | 0,5 | 100 | 50 | 100 | 50 | 100 | | 0 | 0 | 20 | 30 | 30 | 80 | 100 | | | 0 | 0 |
| | 0,125 | 20 | 0 | 80 | 0 | 100 | | 0 | 0 | 0 | 0 | 20 | 30 | 60 | | | 0 | 0 |
| 11 | 4 | 50 | 0 | 100 | 80 | 100 | 100 | 0 | 50 | 20 | 0 | 0 | 80 | 100 | 100 | | 0 | 0 |
| | 1 | 0 | 0 | 100 | 100 | 100 | 80 | 0 | 50 | 0 | 0 | 0 | 80 | 90 | 50 | | 0 | 0 |
| | 0,25 | 0 | 0 | 90 | 0 | 90 | 80 | 0 | | 0 | 0 | 0 | 0 | 80 | 0 | | 0 | 0 |
| 12 | 4 | 30 | 0 | 80 | 60 | 80 | 100 | 0 | 50 | 0 | 0 | 0 | 60 | 80 | 50 | | 0 | 0 |
| | 1 | 30 | 0 | 90 | 90 | 80 | 100 | 0 | | 0 | 0 | 0 | 0 | 20 | | | 0 | 0 |

TABLEAU (III) (suite)

| N°Composé | Doses en kg/ha | PRE-LEVEE | | | | | | | | POST-LEVEE | | | | | | | | |
|---|---|---|---|---|---|---|---|---|---|---|---|---|---|---|---|---|---|---|
| | | ECH | LOL | ABU | IPO | SIN | XAN | BLE | SOJ | ECH | LOL | ABU | IPO | SIN | XAN | | BLE | SOJ |
| 13 | 4 | 90 | 30 | 95 | 0 | 100 | 50 | 0 | 0 | 60 | 30 | 100 | 30 | 100 | 80 | | 0 | 0 |
| | 1 | 60 | 0 | 80 | 0 | 0 | 0 | 0 | | 30 | 30 | 50 | | 100 | 100 | | 0 | 0 |
| | 0,25 | 0 | 0 | 80 | 0 | 0 | 0 | 0 | 0 | 20 | 0 | 20 | 20 | 100 | 50 | | 0 | 0 |
| 14 | 4 | 0 | 0 | 50 | 50 | 80 | 100 | 0 | 100 | 0 | 0 | 30 | 50 | 100 | 100 | | 0 | 0 |
| | 1 | 0 | 0 | 0 | 0 | 30 | 0 | 0 | 0 | 0 | 0 | 0 | 0 | 80 | 100 | | 0 | 0 |
| | 0,25 | 0 | 0 | 0 | 0 | 0 | 0 | 0 | 0 | 0 | 0 | 0 | 0 | 60 | 30 | | 0 | 0 |
| 15 | 4 | 100 | 98 | 100 | 100 | 100 | | 0 | 20 | 100 | 40 | 100 | 100 | 100 | | | 0 | 0 |
| | 1 | 100 | 20 | 95 | 100 | 100 | | 0 | 0 | 100 | 30 | 100 | 100 | 100 | | | 0 | 0 |
| | 0,25 | 30 | 0 | 80 | 20 | 100 | | 0 | 0 | 100 | 0 | 98 | 98 | 100 | | | 0 | 0 |
| 16 | 2 | 20 | 0 | 98 | 0 | 95 | | 0 | 0 | 30 | 0 | 50 | 100 | 100 | | | 20 | 0 |
| | 0,5 | 0 | 0 | 80 | 0 | 0 | | 0 | 0 | 20 | 0 | 30 | 80 | 0 | | | 0 | 0 |
| | 0,125 | 0 | 0 | 50 | 0 | 0 | | 0 | 0 | 0 | 0 | 0 | 80 | 0 | | | 0 | 0 |

TABLEAU (III) (Suite)

| N°Composé | Doses en kg/ha | PRE-LEVEE | | | | | | | | POST-LEVEE | | | | | | | | |
|---|---|---|---|---|---|---|---|---|---|---|---|---|---|---|---|---|---|---|
| | | ECH | LOL | ABU | IPO | SIN | XAN | BLE | SOJ | ECH | LOL | ABU | IPO | SIN | XAN | | BLE | SOJ |
| 17 | 2 | 100 | 100 | 100 | 100 | 100 | | 80 | 0 | 100 | 20 | 100 | 100 | 100 | | | 20 | 0 |
| | 0,5 | 100 | 30 | 100 | 100 | 100 | | 0 | 0 | 98 | 20 | 100 | 100 | 100 | | | 20 | 0 |
| | 0,125 | 0 | 0 | 100 | 0 | 100 | | 0 | 0 | 20 | 0 | 30 | 100 | 100 | | | 0 | 0 |
| 18 | 2 | 100 | 100 | 100 | 100 | 100 | | 100 | 0 | 100 | 30 | 90 | 100 | 100 | | | 20 | 0 |
| | 0,5 | 100 | 100 | 100 | 100 | 100 | | 0 | 0 | 98 | 0 | 20 | 100 | 100 | | | 0 | 0 |
| | 0,125 | 80 | 0 | 98 | 50 | 100 | | 0 | 0 | 20 | 0 | 0 | 90 | 90 | | | 0 | 0 |
| 19 | 4 | 100 | 80 | 100 | 100 | 100 | 80 | 50 | 0 | 100 | 20 | 100 | 100 | 100 | 100 | | 20 | 0 |
| | 1 | 90 | 30 | 100 | 100 | 100 | 0 | 0 | 0 | 90 | 0 | 100 | 100 | 100 | 100 | | 0 | 0 |
| | 0,25 | 30 | 0 | 98 | 50 | 100 | 0 | 0 | 0 | 30 | 0 | 100 | 100 | 100 | 100 | | 0 | 0 |
| 20 | 4 | 100 | 50 | 100 | 100 | 100 | 100 | 0 | 100 | 90 | 20 | 100 | 100 | 100 | 100 | | 0 | 0 |
| | 1 | 90 | 0 | 100 | 100 | 100 | 100 | 0 | 80 | 80 | 20 | 100 | 100 | 100 | 100 | | 0 | 0 |
| | 0,25 | 30 | 0 | 95 | 80 | 100 | 100 | 0 | 100 | 20 | 0 | 80 | 100 | 100 | 100 | | 0 | 0 |

TABLEAU (III) (Suite)

| N° Composé | Doses en kg/ha | PRE-LEVEE | | | | | | | | POST-LEVEE | | | | | | | | |
|---|---|---|---|---|---|---|---|---|---|---|---|---|---|---|---|---|---|---|
| | | ECH | LOL | ABU | IPO | SIN | XAN | BLE | SOJ | ECH | LOL | ABU | IPO | SIN | XAN | | BLE | SOJ |
| 21 | 4 | 80 | 80 | 100 | 50 | 98 | 0 | 0 | 0 | 30 | 20 | 100 | 100 | 100 | 0 | | 0 | 0 |
| | 1 | 70 | 20 | 100 | 50 | 90 | 0 | 0 | 0 | 30 | 0 | 90 | 100 | 100 | 0 | | 0 | 0 |
| | 0,25 | 30 | 0 | 100 | 0 | 30 | 0 | 0 | 0 | 30 | 0 | 90 | 80 | 100 | 0 | | 0 | 0 |
| 22 | 2 | 90 | 20 | 0 | 0 | 100 | | 0 | 0 | 100 | 30 | 80 | 30 | 100 | | | 0 | 0 |
| | 0,5 | 0 | 0 | 0 | 0 | 100 | | 0 | 0 | 100 | 20 | 50 | 30 | 100 | | | 0 | 0 |
| | 0,125 | 0 | 0 | 0 | 0 | 100 | | 0 | 0 | 80 | 0 | 0 | 20 | 100 | | | 0 | 0 |
| 23 | 2 | 90 | 80 | 100 | 0 | 100 | | 0 | 0 | 100 | 40 | 90 | 80 | 100 | | | 20 | 0 |
| | 0,5 | 0 | 0 | 20 | 0 | 100 | | 0 | 0 | 100 | 30 | 40 | 40 | 100 | | | 0 | 0 |
| | 0;125 | 0 | 0 | 0 | 0 | 100 | | 0 | 0 | 20 | 0 | 20 | 0 | 100 | | | 0 | 0 |
| 24 | 4 | 100 | 80 | 100 | 100 | 100 | 80 | 30 | 0 | 90 | 0 | 100 | 100 | 100 | 100 | | 0 | 0 |
| | 1 | 30 | 0 | 60 | 0 | 100 | 0 | 0 | 0 | 30 | 0 | 100 | 50 | 30 | 100 | | 0 | 0 |
| | 0,25 | 0 | 0 | 60 | 0 | 100 | 0 | 0 | 0 | 0 | 0 | 0 | 50 | 20 | 0 | | 0 | 0 |

TABLEAU (III)

| N°Composé | Doses en kg/ha | PRE-LEVEE | | | | | | | | POST-LEVEE | | | | | | | | |
|---|---|---|---|---|---|---|---|---|---|---|---|---|---|---|---|---|---|---|
| | | ECH | LOL | ABU | IPO | SIN | XAN | BLE | SOJ | ECH | LOL | ABU | IPO | SIN | XAN | | BLE | SOJ |
| 25 | 4 | 90 | 80 | 100 | 50 | 100 | 0 | 30 | 0 | 20 | 0 | 30 | 50 | 0 | 0 | | 0 | 0 |
| | 1 | 0 | 0 | 0 | 0 | 95 | 0 | 0 | 0 | 0 | 0 | 0 | 50 | 0 | 0 | | 0 | 0 |
| | 0,25 | 0 | 0 | 0 | 0 | 30 | 0 | 0 | 0 | 0 | 0 | 0 | 30 | 0 | 0 | | 0 | 0 |
| 26 | 4 | 90 | 70 | 100 | 0 | 100 | 0 | 30 | 0 | 20 | 0 | 40 | 50 | 20 | 0 | | 0 | 0 |
| | 1 | 0 | 0 | 95 | 0 | 100 | 0 | 0 | 0 | 0 | 0 | 0 | 0 | 20 | 0 | | 0 | 0 |
| | 0,25 | 0 | 0 | 40 | 0 | 30 | 0 | 0 | 0 | 0 | 0 | 0 | 0 | 20 | 0 | | 0 | 0 |
| 27 | 2 | 100 | 90 | 100 | 100 | 100 | | 50 | 0 | 100 | 0 | 80 | 100 | 100 | | | 0 | 0 |
| | 0,5 | 30 | 0 | 0 | 20 | 100 | | 0 | 0 | 30 | 0 | 30 | 100 | 100 | | | 0 | 0 |
| | 0,125 | 0 | 0 | 0 | 0 | 80 | | 0 | 0 | 20 | 0 | 0 | 100 | 30 | | | 0 | 0 |
| 28 | 2 | 50 | 0 | 100 | 100 | 100 | | 0 | 0 | 40 | 20 | 100 | 100 | | | | 0 | 0 |
| | 0,5 | 0 | 0 | 50 | 0 | 20 | | 0 | 0 | 30 | 0 | 100 | 100 | | | | 0 | 0 |
| | 0,125 | 0 | 0 | 0 | 0 | 0 | | 0 | 0 | 20 | 0 | 100 | 100 | | | | 0 | 0 |

TABLEAU (III)

| N°Composé | Doses en kg/ha | PRE-LEVEE | | | | | | | | POST-LEVEE | | | | | | | | |
|---|---|---|---|---|---|---|---|---|---|---|---|---|---|---|---|---|---|---|
| | | ECH | LOL | ABU | IPO | SIN | XAN | BLE | SOJ | ECH | LOL | ABU | IPO | SIN | XAN | | BLE | SOJ |
| 29 | 2 | 70 | 50 | 100 | 70 | 100 | | 10 | 0 | 30 | 0 | 50 | 100 | 100 | | | 10 | 0 |
| | 0,5 | 30 | 30 | 90 | 0 | 100 | | 0 | 0 | 10 | 0 | 30 | 90 | 100 | | | 0 | 0 |
| | 0,125 | 0 | 0 | 70 | 0 | 100 | | 0 | 0 | 0 | 0 | 10 | 100 | 80 | | | 0 | 0 |
| 30 | 2 | 60 | 60 | 100 | 100 | 100 | | 10 | 0 | 40 | 20 | 60 | 100 | 100 | | | 0 | 0 |
| | 0,5 | 30 | 30 | 100 | 70 | 100 | | 0 | 0 | 20 | 0 | 20 | 90 | 90 | | | 10 | 0 |
| | 0,125 | 30 | 30 | 100 | 40 | 100 | | 0 | 0 | 20 | 0 | 0 | 50 | 50 | | | 0 | 0 |

AGROCHIMIE
Siège Social
Rue Pierre Baizet
69009 LYON
FRANCE

Charles BRACHOTTE

## REVENDICATIONS

1) Produits caractérisés en ce qu'ils ont pour formule :

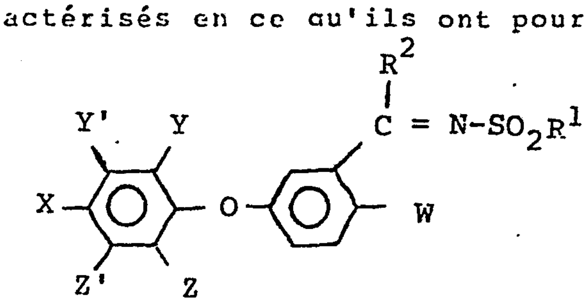

dans laquelle :

- W, Y, Y', X, Z', Z représentent l'atome d'hydrogène ou un atome d'halogène ou un groupe $NO_2$ ou CN, ou un groupe polyhaloalkyle, ou un groupe alkyle, ou alkoxyle, les divers groupes alkyle ou alcoxyle précités, ayant le plus souvent de 1 à 4 atomes de carbone,
- $R^1$ représente un groupe alkyle, alcényle, alcynyle ou phényle, éventuellement substitué,
- $R^2$ représente un atome d'halogène ou un groupe CN ou SCN ou $OR^3$ ou $SR^4$ ou $NR^5R^6$,
- $R^3$ représente un groupe alkyle éventuellement substitué ou un groupe allyle ou propargyle ou alkylcarbonyle ou alkylidène imino ou succinimido.
- $R^4$ représente l'atome d'hydrogène ou un cation ou a une des significations données pour $R^3$,
- $R^5$ représente l'atome d'hydrogène ou un groupe alkyle éventuellement substitué ou un groupe alkylcarbonyle ou alkyl sulfonyle,

- $R^6$ a l'une ces significations données pour $R^5$ ou représente un cation ou un groupe CN ou un groupe $OR^{10}$, $R^{10}$ étant l'atome d'hydrogène ou un cation, ou un groupe alkyle ou carboxylate ou carboxamide ou alkylcarbonyle.

2) Produits selon la revendication 1, caractérisés en ce que

- W représente l'atome d'hydrogène ou un atome d'halogène, notamment Cl, Br, F ou un groupe $NO_2$ ou CN,
- Y représente l'atome d'hydrogène ou un atome d'halogène, notamment Cl, Br, F ou un groupe $NO_2$ ou CN ou $CF_3$ ou $CH_3$,
- Y', Z', Z représentent l'atome d'hydrogène ou unatome d'halogène, notamment Cl, Br ou F,
- X représente un atome d'halogène, notamment Cl, Br, F ou un groupe $NO_2$ ou $CF_3$ ou $CH_3$ ou $C_2H_5$,
- $R^1$ représente :
  . un groupe alkyle, de 1 à 12 atomes de carbone, éventuellement substitué par :
    - un ou plusieurs atomes d'halogènes, notamment Cl, Br, F, ou par
    - un ou plusieurs groupes alkoxyle ou alkylthio ayant le plus souvent de 1 à 4 atomes de carbone, ou par
    - un ou plusieurs groupe CN, ou par
    - un groupe phényle, lui-même éventuellement substitué, notamment par un plusieurs atomes d'halogène, ou
  . un groupe alcényle ou alcynyle ayant le plus souvent de 2 à 4 atomes de carbone, notamment un radical vinyle, éthynyle, allyle, propargyle, ou
  . un groupe phényle, lui-même éventuellement substitué, notamment par un ou plusieurs atomes d'halogène, ou groupes nitro, ou radicaux alkyle ayant le plus souvent de 1 à 4 atomes de carbone.

- $R^2$ représente un atome d'halogène, de préférence le chlore ou un groupe CN ou SCN, ou un groupe $OR^3$ ou $SR^4$ ou $NR^5R^6$.
- $R^3$ représente :
  . un groupe alkyle, ayant le plus souvent de 1 à 4 atomes de carbone, et éventuellement substitué par :
    - un ou plusieurs atomes d'halogène, notamment Cl, Br, F ou par
    - un ou plusieurs groupes alkonyle ou alkylthio ayant le plus souvent de 1 à 4 atomes de carbone, ou par
    - un ou plusieurs groupes $NO_2$ ou CN, ou par
    - un groupe carboxyle ou l'un de ses dérivés de type sel, ester ou amide, notamment un groupe $COOR^7$, $R^7$ étant l'atome d'hydrogène ou un groupe alkyle ayant le plus souvent de 1 à 4 atomes de carbone ou un cation métallique ou ammonium, ou par
    - un groupe alkylcarbonyle, notamment acétyle, ou par
    - un groupe phényle, lui-même éventuellement substitué, notamment par un ou plusieurs atomes d'halogène, ou par
    - un groupe 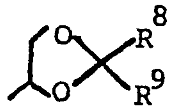 , $R^8$ et $R^9$, identiques ou différents étant l'atome d'hydrogène ou un groupe alkyle ayant le plus souvent de 1 à 4 atomes de carbone, ou
  . un groupe allyle ou propargyle, ou

. un groupe alkylcarbonyle, notamment acétyle, ou

. un groupe 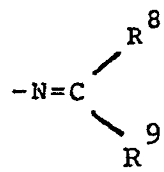 ou 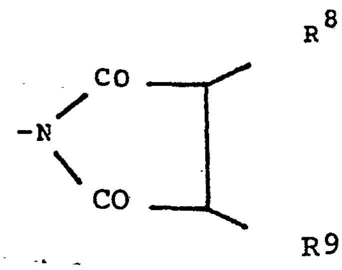

$R^8$ et $R^9$ ayant l'une des significations déjà indiquées.

- $R^4$ représente l'atome d'hydrogène, ou un cation de métal alcalin ou ammonium, ou a l'une des significations données pour $R^3$,

- $R^5$ représente :

. l'atome d'hydrogène, ou

. un groupe alkyle, ayant le plus souvent de 1 à 4 atomes de carbone, et éventuellement substitué par un groupe carboxyle ou l'un de ses dérivés de type sel, ester, ou amide, notamment un groupe $COOR^7$, $R^7$ ayant l'une des significations déjà indiquées,

ou

. un groupe alkylcarbonyle ou alkylsulfonyle, notamment acétyle ou méthanesulfonyle.

- $R^6$ a l'une des significations données pour $R^5$, ou représente un cation de métal alcalin ou ammonium, ou un groupe CN ou un groupe $OR^{10}$, $R^{10}$ étant :

- l'atome d'hydrogène, ou
- un cation de métal alcalin ou ammonium, ou
- un groupe alkyle, ayant le plus souvent de 1 à 4 atomes de carbone, et éventuellement substitué par un groupe carboxyle ou l'un de ses dérivés de type sel, ester, ou amide, notamment un groupe $COOR^7$, $R^7$ ayant l'une des significations déjà indiquées, ou

- un groupe $COOR^7$ ou $CONHR^7$, $R^7$ ayant l'une des significations déjà indiquées, ou
- un groupe alkylcarbonyle, notamment acétyle.

3) Produits selon l'une des revendications 1 ou 2, caractérisés en ce que X est Cl ou $CF_3$, Y' et Z' sont H, Y est Cl, Z est Cl ou F ou H, W est $NO_2$.

4) Produits selon l'un des revendications 1 à 3, caractérisés en ce que $R^2$ est Cl ou un radical $OR^3$ ou $SR^3$ ou $NR^5R^6$ ; $R^1$, $R^3$ et $R^6$ sont des groupes alkyle ; $R^4$ et $R^5$ sont l'atome d'hydrogène ou un groupe méthane sulfonyle ; $R^6$ est l'atome d'hydrogène ou un cation de métal alcalin ou d'ammonium.

5) Produits selon l'une des revendications 1 à 4, caractérisés en ce que X est $CF_3$, Z est H, $R^2$ est alkoxy.

6) Produits selon l'une des revendications 1 à 5, caractérisés en ce que $R^2$ est SH, les produits étant sous une forme tautomère.

7) Procédé de préparation de produits selon l'une des revendications 1 à 6 dans la formule (I) desquels $R^2$ est un atome d'halogène, caractérisé en ce que l'on fait réagir entre -30 et 150°C et en milieu solvant un agent d'halogènation sur un composé de formule :

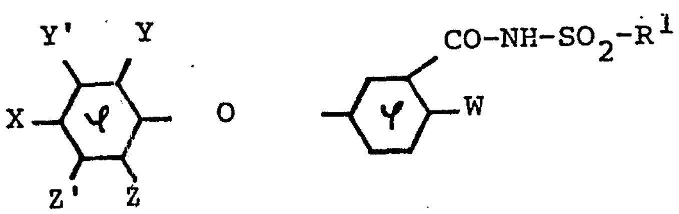

(II)

dans laquelle les divers substituants ont la même signification que dans la formule (I).

8) Procédé selon la revendication 7, caractérisé en ce que l'agent d'halogènation est choisi dans le groupe constitué par $PCl_3$, $PCl_5$, $POCl_3$, $SOCl_2$, $COCl_2$, $SO_2Cl_2$.

9) Procédé de préparation de produits selon l'une des revendications 1 à 6 dans la formule (I) desquels $R^2$ a une autre signification que l'atome d'halogène, caractérisé en ce que l'on fait réagir entre 0 et 150°C et en milieu solvant un halogène d'imidoyle de formule :

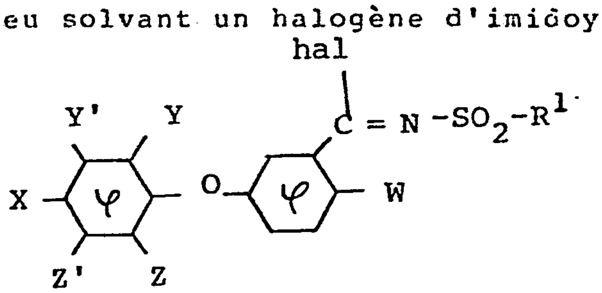

(III)

dans laquelle les divers substituants ont la même signification que dans la formule (I) des revendications 1 à 6 et hal représente un atome d'halogène, de préférence le chlore,

avec un composé de formule $R^2H$, $R^2$ ayant la même signification que dans la formule (I) sauf halogène.

10) Procédé de préparation de produits selon l'une des revendications 1 à 5 dans la formule (I) desquels $R^2$ est un groupe $OR^3$ ou $SR^4$ ou $NR^5R^6$, caractérisé en ce que l'on fait réagir un halogènure d'imidoyle de formule :

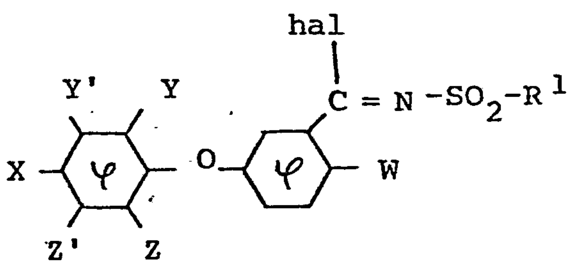

(III)

dans laquelle les divers substituants ont la même signification que dans la formule (I) et hal est un atome d'halogène avec un composé de formule $R^3OM$, ou $R^4SM$ ou $R^5R^6NM$, M étant un atome d'hydrogène ou un atome de métal alcalin.

11) Procédé selon la revendication 10), caractérisé en ce que $R^2$ est $OR^3$ et que la réaction s'effectue entre 10 et 150°C dans un composé de formule $R^3OH$ comme solvant, M étant un atome de sodium ou de potassium.

12) Compositions herbicides, caractérisées en ce qu'elles contiennent comme matière active un produit selon l'une des revendications 1 à 6, cette matière active étant en association avec au moins un support inerte, usuel, acceptable en agriculture.

13) Compositions selon la revendication 12), caractérisées en ce qu'elles contiennent 0,5 à 95 % de matière active.

14) Compositions selon la revendication 13), caractérisées en ce qu'elles contiennent 1 à 95 % de support et 0,1 à 20 % d'agent tensioactif.

15) Procédé pour le désherbage sélectif des cultures, caractérisé en ce qu'on applique une dose efficace d'une matière activeselon l'une des revendications 1 à 6.

16) Procédé selon la revendication 15), caractérisé en ce qu'on applique une composition selon l'une des revendications 12) à 14), la matière active étant appliquée à raison de 0,01 à 5 kg/ha, de préférence de 0,1 à 2 kg/ha.

17) Procédé selon l'une des revendications 14) à 16), caractérisé en ce que la culture est le soja ou une céréale notamment le maïs, le tournesol, le cotonnier ou le haricot.

RHONE-POULENC
AGROCHIMIE
Siège Social
14/20, Rue Pierre Baizet
69009 LYON
FRANCE

Charles BRACHOTTE